Europäisches Patentamt

European Patent Office

Office européen des brevets

(19)

(11) Veröffentlichungsnummer: **0 098 411**
**B2**

(12) **NEUE EUROPÄISCHE PATENTSCHRIFT**

(45) Veröffentlichungstag der neuen Patentschrift:
05.09.90

(51) Int. Cl.⁵: **A 61 B 5/02,** A 61 B 19/00

(21) Anmeldenummer: 83105757.5

(22) Anmeldetag: 11.06.83

(54) Belüftungsfilter.

(30) Priorität: 15.06.82 DE 8217016 u

(43) Veröffentlichungstag der Anmeldung:
18.01.84 Patentblatt 84/03

(45) Bekanntmachung des Hinweises auf die
Patenterteilung:
09.09.87 Patenblatt 87/37

(45) Bekanntmachung des Hinweises auf die
Entscheidung u¨ber den Einspruch:
05.09.90 Patentblatt 90/36

(84) Benannte Vertragsstaaten:
AT BE CH DE FR GB IT LI LU NL SE

(56) Entgegenhaltungen:
DE-A-2 730 911        US-A-3 783 895
DE-A-2 818 146        US-A-4 177 149
DE-A-2 851 838        US-A-4 190 426
GB-A-2 000 685        US-A-4 237 879
US-A-3 435 819        US-A-4 271 973

"Sterile Dosage Forms", S.Turco and R.E.King,
2nd Edition, 1979,

(73) Patentinhaber: B. Braun-SSC AG
Gerliswilstrasse 74
CH-6020 Emmenbrücke (CH)

(72) Erfinder: Lesemann, Egon
Rhönstrasse 3
D-3501 Körle (DE)

(74) Vertreter: von Kreisler, Alek, Dipl.-Chem. et al
Patentanwälte Von Kreisler-Selting-Werner
Deichmannhaus am Hauptbahnhof
D-5000 Köln 1 (DE)

EP 0 098 411 B2

Courier Press, Leamington Spa, England.

**Beschreibung**

Die Erfindung bezieht sich auf einen Belüftungsfilter an dem offenen Ende des Meßschenkels eines Venendruckmeßgerätes, bestehend aus einem an beiden Enden offenen Gehäuse, dessen eines Ende über einen Anschlußteil mit dem Meßschenkel verbunden ist und in dessen lichtem Querschnitt Filtermaterial angeordnet ist.

Venendruckmeßgeräte besitzen im allgemeinen einen Meßschenkel, der auf einer graduierten Meßleiste befestigt ist. Die Flüssigkeitssäule in dem Meßschenkel wird vom jeweiligen Venendruck bestimmt. Dadurch ist es erforderlich, daß der Meßschenkel, der gewöhnlich aus einem PVC-Schlauch besteht, am oberen Ende offen sein muß.

Diese Öffnung wird üblicherweise mit einem Luftfilter verschlossen. Bei dem eingangs erwähnten bekannten Belüftungsfilter (US—A—3 435 819) enthält das Gehäuse Filtermaterial, das einen lose gestopften Propfen bildet, der keine definierte Porengröße aufweist und nicht hydrophob ist. In das Gehäuse ragt ein Ende eines Schlauchstückes hinein, das unlösbar mit dem Gehäuseboden verbunden ist. Das andere Ende des Schlauchstückes trägt eine Kappe, die auf einen Manometerzylinder aufgesteckt ist und Teil eines Schwimmerventils bildet, das das Ausströmen von Infusionsflüssigkeit aus dem Manometerzylinder zu dem Belüftungsfilter verhindern soll. Der ganze Aufbau ist infolge des Schlauchstückes und des Schwimmerventils unhandlich sperrig und für einen Wegwerfteil zu teuer. Außerdem können Undichtigkeiten an den Verbindungsstellen Kontaminationen im Innern des Venendruckmeßgerätes und außerhalb desselben verursachen, die für den Patienten und das Bedienungspersonal gefährlich sein können.

Ferner ist ein Verschlußteil für ein Gerät bekannt (DE—A—2 818 146), der eine dicke keimdichte und hydrophobe Filterscheibe aufweist, die mittels eines abnehmbaren Befestigungskörpers an einem Stutzen des Gerätes gehalten ist. Diese Anordnung dient der Bewahrung der Sterilität des Gerätes im Zeitraum zwischen Sterilisation und Anwendung. Zur Verwendung bei einem Venendruckmeßgerät ist sie ungeeignet, weil die dicke Filterscheibe und der Befestigungskörper klobig sind und günstige Anschlußmöglichkeiten zur Verbindung mit einem Venendruckmeßgerät-Meßschenkel fehlen.

Ausgehend von dem eingangs erwähnten Stand der Technik liegt der Erfindung die Aufgabe zugrunde, einen Belüftungsfilter so auszubilden, daß er als gedrungen gestaltetes, preiswertes Wegwerfteil eine sichere Anwendung eines Venendruckmeßgerätes gewährleistet.

Diese Aufgabe wird erfindungsgemäß dadurch gelöst, daß das Filtermaterial eine bakteriendichte und hydrophobe flache Filterscheibe mit einer Porengröße zwischen 0,2 und 1,2 µm, vorzugsweise 0,2 und 0,45 µm, ist, und daß der Anschlußteil als Außenkonus für eine lösbare Steckverbindung ausgebildet ist.

Auf diese Weise erhält man einen Belüftungsfilter, der folgende Vorteile hat:
1. Bakterielle Kontamination der Infusionslösung (Bakterienretention) wird durch die spezielle Porengröße verhindert,
2. der Austritt von Infusionslösung beim Füllen des Meßschenkels wird durch die hydrophobe Beschaffenheit des Filtermaterials unterbunden,
3. die Verwendung einer flachen Filterscheibe verringert die Bauhöhe des Belüftungsfilters,
4. durch die Ausbildung des Anschlußteiles als Außenkonus ist es möglich, den Belüftungsfilter mit dem offenen Ende des Meßschenkels durch einfaches Zusammenstecken luftdicht zu verbinden. Durch Auseinanderziehen der Steckverbindung kann der Belüftungsfilter vom Meßschenkel leicht abgenommen werden. Seine preiswerte Herstellbarkeit rechtfetigt sein Wegwerfen nach einmaliger Benutzung.

Durch den Einsatz des erfindungsgemäßen Belüftungsfilters werden Behältnisse und Leitungssysteme, in denen sich dem Patienten zu applizierende oder direkt mit diesem in Verbindung stehende Lösungen befinden, sicherer, und zwar sowohl für den Patienten als auch für das Bedienungspersonal.

Der Filterkörper besteht vorteilhafterweise aus physiologisch indifferentem Werkstoff.

Der als flache Scheibe ausgebildete Filterkörper kann in einem Gehäuse unterschiedlicher Konstruktion je nach Größenbedarf der Filterfläche durch Einschweißen, Klemmen oder auf andere Weise den Erfordernissen des Grundmaterials entsprechend fixiert sein.

Zweckmäßigerweise ist vorgesehen, daß die Filterscheibe mittels eines Klemmteils in dem Gehäuse befestigt ist, der aus einer zylindrischen Kappe mit gelochtem Boden besteht, die passend in ein Ende des Gehäuses eingesetzt ist und daß der Filterkörper zwischen dem Rand der Kappe und einem Rand des Gehäuses eingeklemmt angeordnet ist.

In der Zeichnung sind Ausführungsbeispiele der Erfindung schematisch dargestellt.

Figur 1 zeigt das Prinzip eines üblichen Venendruckmeßgerätes

Figur 2 ist ein Ausführungsform des Gehäuses mit Filterkörper und

Figur 3 stellt eine andere Ausführungsform eines Gehäuses mit Filterkörper dar.

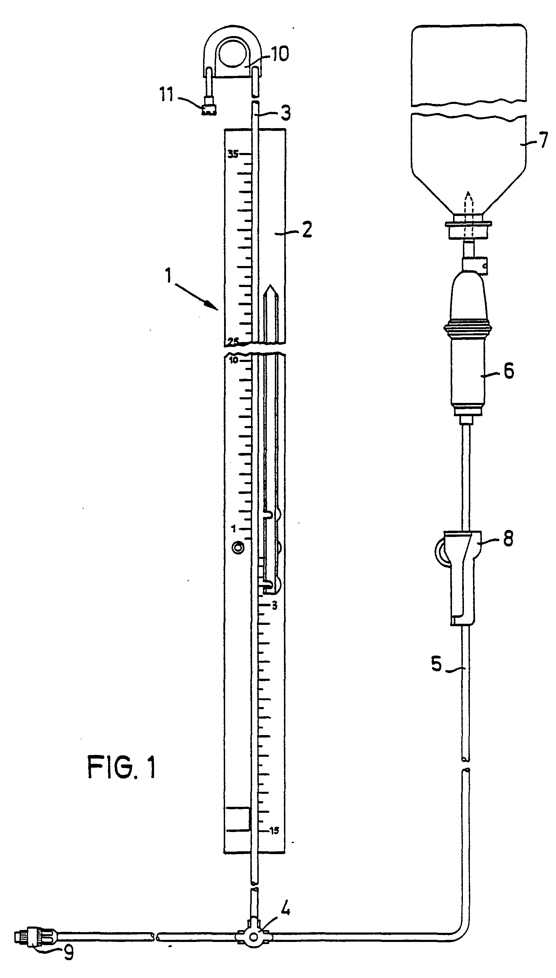

Das in Fig. 1 gezeigte Venendruckmeßgerät besteht aus einem Meßschenkel 1, der einen Schlauch 3 aufweist, welcher auf einer graduierten Meßleiste 2 befestigt ist. Das untere Ende des Schlauches 3 ist über ein Kupplungsstück 4 an einen Schlauch 5 angeschlossen, der über eine Tropfkammer 6 mit einem Behälter 7 für Infusionslösung 0. dgl. in Verbindung steht. Positionsziffer 8 bezeichnet eine Rollenklemme, die an den Schlauch 5 angesetzt ist. Ein Anschlußstück 9 stellt die Verbindung zum Patienten her. Das obere Ende des Schlauches 3 ist über ein Umlenkteil 10 herumgelegt, so daß das offene Ende 11 des Schlauches 3 nach unten gerichtet ist. Das offene Ende 11 kann ein Paßstück mit Innenkonus aufweisen.

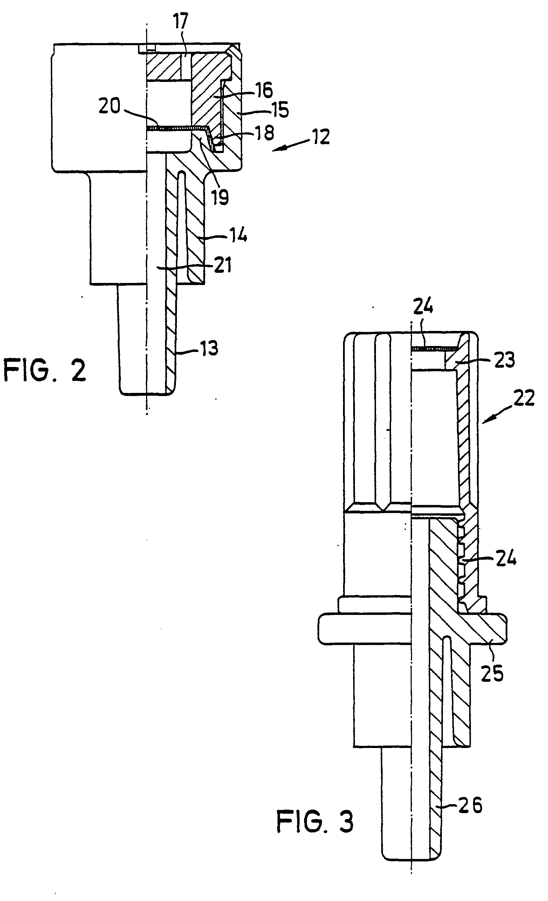

Zum Verschluß des offenen Endes 11 des Schlauches 3 des Meßschenkels 1 dienen die in den Fig. 2 und 3 dargestellten Belüftungsfilter.

Der Belüftungsfilter gemäß Fig. 2 weist ein zylindrisches, hülsenförmiges Gehäuse 12 auf, dessen eines Ende mit einem Außenkonus 13 und einem diesen auf einem Teil seiner Länge umgebenden Rohrstück 14 ausgestattet ist. Das andere Ende des Gehäuses 12 trägt eine Erweiterung 15, in die eine Kappe 16 passend eingesetzt ist. Der Boden der Kappe 16 ist mit Löchern 17 versehen und sein unterer Rand 18 ist so profiliert, daß er einen geschlossenen Ringsteg 19 in der Erweiterung 15 passend übergreift. Zwischen den Ringsteg 19 und den Rand 18 ist eine Filterscheibe 20 so eingeklemmt, daß ihr Rand nach unten abgewinkelt ist. Die Filterscheibe 20 wird auf diese Weise in den Kanal 21 des Gehäuses 12 ein gespannt und durch die Ausbildung der Filterscheibe 20 als bakteriendichter und hydrophober Filterkörper mit definierter Porengröße verhindert sie den Flüssigkeitsaustritt und eine Bakterienretention, wenn der Anschlußkonus 13 in das offene Ende 11 des Schlauches 3 des Meßschenkels 1 eingesetzt ist.

Eine andere Ausführungsmöglichkeit des Belüftungsfilters zeigt Fig. 3. In das offene Ende eines Gehäuses 22 ist auf einen nach innen gerichteten Ringflansch 23 ein bakteriendichter und hydrophober Filterkörper in Form einer Filterscheibe 24 aufgeschweißt. Das Gehäuse 22 ist als zylindrische Hülse gestaltet, deren der Filterscheibe 22 abgewandtes Ende mittels einer Abdichtprofilierung 24 in einem Ansatz auf ein Zwischenstück 25 passend aufgesteckt ist. Das Zwischenstück 25 weist einen Außenkonus 26 zum Einsetzen in das offene Ende 11 des Schlauches 3 des Meßschenkels 1 auf. Der Außenkonus 26 ist auf einem Teil seiner Länge von einem Rohrstück 14 umgeben. Auch in diesem Falle eliminiert der bakteriendichte und hydrophobe Filterkörper 24 mit definierter Porengröße die Gefahr einer bakteriellen Kontamination der Infusionslösung aus dem Behälter 7 und verhindert das versehentliche Austreten von Infusionslösung beim Füllen aus der Öffnung 11 des Schlauches 3.

**Patentansprüche**

1. Belüftungsfilter an dem offenen Ende des Meßschenkels eines Venendruckmeßgerätes, bestehend aus einem an beiden Enden offenen Gehäuse, dessen eines Ende über einen Anschlußteil mit dem Meßschenkel verbunden ist und in dessen lichtem Querschnitt Filtermaterial angeordnet ist, dadurch gekennzeichnet, daß das Filtermaterial eine bakteriendichte und hydrophobe flache Filterscheibe (20; 24) mit einer Porengröße zwischen 0,2 und 1,2 µm, vorzugsweise zwischen 0,2 und 0,45 µm, ist, und daß der Anschlußteil als Außenkonus (13; 26) für eine lösbare Steckverbindung ausgebildet ist.

2. Belüftungsfilter nach Anspruch 1, dadurch gekennzeichnet, daß die Filterscheibe (20, 24) aus physiologisch indifferentem Werkstoff besteht.

3. Belüftungsfilter nach Anspruch 1 oder 2, dadurch gekennzeichnet, daß die Filterscheibe (20; 24) mittels eines Klemmteiles in dem Gehäuse (12) befestigt ist, der aus einer zylindrischen Kappe (16) mit gelochtem Boden besteht, die passend in ein Ende des Gehäuses (12) eingesetzt ist, und daß der Filterkörper (20) zwischen dem Rand (18) der Kappe (16) und einem Rand (19) des gehäuses (12) eingeklemmt angeordnet ist.

4. Belüftungsfilter nach Anspruch 1 oder 2, dadurch gekennzeichnet, daß die Filterscheibe (24) mit dem Gehäuse (22) durch Anschweißen verbunden ist.

**Revendications**

1. Filtre d'aération placé sur l'extrémité ouverte de la branche de mesure d'un appareil de mesure de la tension veineuse, constitué par un boîtier ouvert aux deux extrémités et dont une extrémité est reliée par l'intermédiaire d'un organe de raccordement à la branche de mesure et dans la section transversale dégagée duquel se trouve disposé un matériau filtrant, caractérisé en ce que le matériau filtrant est un disque filtrant plat, étanche aux bactéries et hydrophobe (20; 24), possédant des pores d'une taille comprise entre 0,2 et 1,2 µm et de préférence entre 0,2 et 0,45 µm, et en ce que l'organe de raccordement est réalisé sous la forme d'un cône extérieur (13; 26) pour un connecteur enfichable détachable.

2. Filtre d'aération selon la revendication 1, caractérisé en ce que le disque filtrant (20; 24) est réalisé en une substance indifférente du point de vue physiologique.

3. Filtre d'aération selon la revendication 1 ou 2, caractérisé en ce que ledit disque filtrant (20; 24) est fixé au moyen d'un organe de serrage dans le boîtier (12) qui est constitué par un capuchon cylindrique (16) muni d'un fond perforé, qui est inséré de façon adaptée dans une extrémité du boîtier (12), et que le corps filtrant (20) est disposé en étant serré entre le bord (18) du capuchon (16) et un bord (19) du boîtier (12).

4. Filtre d'aération selon la revendication 1 ou 2, caractérisé en ce que le disque filtrant (24) est relié par soudage au boîtier (22).

**Claims**

1. Ventilation filter at the open end of the measuring leg of a vein pressure meter comprising a housing open at both ends of which one end is joined to the measuring leg via a connecting member, and filter material being provided in its clear cross section, characterized in that the filter material is a bacteria-tight and hydrophobic flat filter disk (20; 24) having a pore size between 0.2 and 1.2 µm, preferably between 0.2 and 0.45 µm, and that the connecting member is an external cone (13; 26) designed for a detachable plug connection.

2. Ventilation filter as defined in claim 1, characterized in that the filter disk (20; 24) is made of a physiologically indifferent material.

3. Ventilation filter as defined in claim 1 or 2, characterized in that the filter disk (20; 24) is secured in the housing (12) by a clamping member which consists of a cylindrical cap (16) having a perforated bottom and being fitted in one end of the housing (12) and that the filter body (20) is clampingly arranged between the edge (18) of clamp (16) and one edge (19) of housing (12).

4. Ventilation filter as defined in claim 1 or 2, characterized in that the filter disk (24) is connected by welding to housing (22).

EP  0 098 411  B2

FIG. 1

FIG. 2

FIG. 3